# EUROPÄISCHE PATENTANMELDUNG

(11) **EP 0 310 545 A2**
(43) Veröffentlichungstag der Anmeldung: **05.04.1989**
(21) Anmeldenummer: 88810582.2
(22) Anmeldetag: 24.08.1988
(51) Int. Cl.: C08G 59/40, C08G 59/68, C08J 5/24

(54) **Cyanoguanidine als Härter für Epoxidharze**

(30) Priorität: 02.09.1987 CH 3359/87
(71) Anmelder: CIBA-GEIGY AG, 4002 Basel (CH)
(72) Erfinder: Flury, Peter, Dr., CH-4204 Himmelried (CH); Alder, Alex, Dr., CH-4422 Arisdorf (CH)

(57) **Zusammenfassung**

Härtbare Epoxidharz-Stoffgemische enthaltend Cyanoguanidine der Formel I 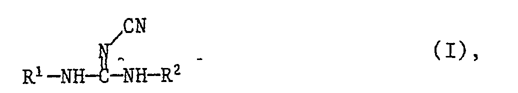 worin zum Beispiel R¹ und R² Phenyl bedeuten, als latente Härter zeichnen sich durch gute Eigenschaften aus und sind besonderss gut geeignet für den Einsatz als Laminierharze.

## Beschreibung

Die Erfindung betrifft härtbare Epoxidharz-Stoffgemische enthaltend bestimmte Cyanoguanidine als latente Härter sowie deren Verwendung.

Dicyandiamid hat sich als latenter Härter für Epoxidharze seit langem bewährt (vgl. z.B. H. Lee und K. Neville "Handbook of Epoxy Resins", McGraw Hill, New York, 1982, Seite 10-16) und wird in der Praxis vor allem als Härter für feste Laminierharze verwendet. Dicyandiamid hat aber den gravierenden Nachteil, dass es nur in für das Laminiergeschäft ungeeigneten Lösungsmitteln, wie Wasser, Aceton-Wasser, Methanol, N-Methylpyrrolidon, Dimethylformamid, Hydroxylgruppen enthaltenden Ethern usw. löslich ist. Das heute üblicherweise verwendete Lösungsmittel, 2-Methoxyethanol, ist aus toxikologischen Gründen bedenklich.

Die Cyanoguanidine der erfindungsgemässen Stoffgemische sind, wie Dicyandiamid, latente Härter, welche bei Raumtemperatur stabil sind, während sie bei erhöhter Temperatur eine schnelle Vernetzung der Harze verursachen. Sie sind in für die Applikation von Epoxidharzen geeigneten, unproblematischen Lösungsmitteln gut löslich.

Gegenstand der Erfindung sind härtbare Stoffgemische enthaltend
(a) ein Epoxidharz und
(b) als Härter für das Epoxidharz ein Cyanoguanidin der Formel I 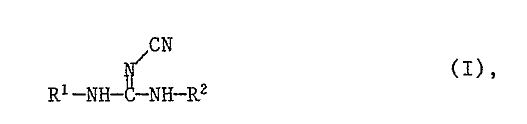 worin R¹ und R² unabhängig voneinander C₁-C₁₂-Alkyl, C₂-C₁₂-Alkenyl, C₅-C₁₀-Cycloalkyl, C₆-C₁₀-Aryl, C₇-C₁₂-Aralkyl sind, oder einen C₄-C₈-heterocyclischen Rest oder eine Gruppe der Formel II bedeuten 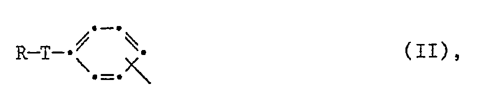 worin R für Phenyl oder für einen C₄-C₅-heterocyclischen Rest und T für Methylen, Isopropyliden, CO, O, S oder SO₂ stehen, wobei die Reste R¹ und R² unsubstituiert sind oder C₁-C₄-Alkyl, C₁-C₄-Alkoxy, Nitro, Halogen,

   R³-O- 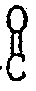 - oder

   R³- 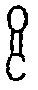 -O- Substituenten enthalten und R³ Phenyl oder C₁-C₄-Alkyl bedeutet.

Die Struktur der in den erfindungsgemässen Stoffgemischen enthaltenen Cyanoguanidine wurde der Einfachheit halber als 2-Cyanoguanidin (d.h. mit der Cyanogruppe am =N-Stickstoffatom gebunden) dargestellt. Es versteht sich von selbst, dass diese Verbindungen auch als tautomere Formen, d.h. als 1- bzw. 3-Cyanoguanidine, vorliegen können und dass die Lage des Gleichgewichts zwischen den möglichen Tautomeren von den Resten R¹ und R² abhängt.

Cyanoguanidine des Typs 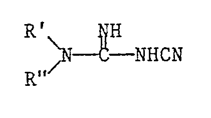 sind als Härter für Acetalharze oder für Polyurethanharze z.B. in der JP-OS 85/44543 bzw. in der US 3,864,313 beschrieben. Die in der erwähnten JP-OS beschriebenen härtbaren Acetalharz-Stoffgemische enthalten zur Verbesserung der Haftung des Polyacetalharzes und der darin enthaltenen anorganischen Füllstoffe noch kleine Mengen eines Polyglycidylethers oder eines Diglycidylesters.

Die JP-OS 86/207425 offenbart die Verwendung von Gemischen aus Cyanoguanidinen, insbesondere Dicyandiamid, Polyether-Polyaminen und substituierten Guanidinen als Härter für spezielle Elastomer modifizierte Epoxidharze. Diese Härtergemische eignen sich nicht als latente Härter.

Die Cyanoguanidine der Formel I können durch Umsetzung eines Carbodiimids der Formel III

R¹-N=C=N-R² (III),

worin R¹ und R² die oben angegebene Bedeutung haben, mit Cyanamid hergestellt werden.

Die Reste R¹ und R² der Cyanoguanidine der Formel I können unabhängig voneinander eine geradkettige oder verzweigte Alkylgruppe mit 1-12, vorzugsweise 1-6 und insbesondere 3 oder 4 C-Atomen sein. Beispiele für geeignete Alkylgruppen sind Dodecyl, Decyl, Octyl, Heptyl, Butyl, Propyl, Ethyl oder Methyl.

Wenn R¹ und/oder R² Alkenylgruppen mit 2-12 C-Atomen bedeuten, können diese ebenso geradkettig oder verzweigt sein, und die Doppelbindung kann in jeder der möglichen Stellungen sein. Bevorzugt werden Alkenylgruppen mit 2-6, insbesondere 3-4 C-Atomen, wie z.B. 1-, 2- oder 3-Butenyl oder 1- oder 2-Propenyl.

Wenn R¹ und/oder R² einen Cycloalkylrest bedeuten, handelt es sich vorzugsweise um Cyclopentyl oder insbesondere Cyclohexyl. Die Cycloalkylreste können auch mehr als einen Ring enthalten, wie z.B. Decalinyl.

Wenn R¹ und/oder R² einen Arylrest bedeuten, so handelt es sich vorzugsweise um Phenyl, substituiertes Phenyl oder Naphthyl. Als C₇-C₁₂-Aralkyl kommen z.B. Benzyl oder Naphthylmethyl in Frage.

Wenn R¹ und/oder R² einen heterocyclischen Rest bedeuten, handelt es sich vorzugsweise um 5- oder 6-gliedrige Ringe mit einem oder zwei Heteroatomen, welche vorzugsweise N, O oder S sind, wie z.B. einwertige Reste des Furans, Pyrans, Pyridins, Pyrrols, Imidazols, Thiophens usw.

Bedeuten die Reste R¹ und/oder R² eine Gruppe der Formel II, so ist R Phenyl oder ein heterocyclischer Rest, wie z.B. einer der oben genannten heterocyclischen Reste. Bevorzugt bedeutet T Methylen, Isopropyliden oder insbesondere O. Der Phenylenrest in der Gruppe der Formel II ist vorzugsweist 1,4-Phenylen.

Alle oben erwähnten Reste R¹ und/oder R² können unsubstituiert sein oder einen oder mehrere, vorzugsweise einen oder zwei, der erwähnten Substituenten enthalten. Geeignete C₁-C₄-Alkylsubstituenten sind Methyl, Ethyl, n-Propyl, iso-Propyl, n-, iso-, sec.- oder tert.-Butyl. Geeignete C₁-C₄-Alkoxysubstituenten sind solche, welche die gerade erwähnten Alkylreste enthalten. Geeignete Halogensubstituenten sind z.B. Iod, Brom, Fluor und insbesondere Chlor. Geeignete Alkoxycarbonyl oder Alkylcarboxyreste

R³-O- 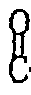 - oder

R³- 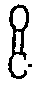 -O- sind solche, worin R³ Phenyl oder eine der oben erwähnten C₁-C₄-Alkylgruppen, insbesondere Methyl, darstellt.

Bevorzugt werden Cyanoguanidine eingesetzt, worin R¹ und R² unabhängig voneinander C₁-C₆-Alkyl, C₂-C₆-Alkenyl, C₅-C₆-Cycloalkyl, C₆-C₁₀-Aryl, Benzyl oder einen Rest der Formel II bedeuten.

Besonders bevorzugt werden Cyanoguanidine, worin R¹ und R² unabhängig voneinander C₃-C₄-Alkyl, C₃-C₄-Alkenyl, Cyclohexyl, Phenyl, Tolyl, Methoxyphenyl, Ethoxyphenyl, Dichlorphenyl, Benzyl, Naphthyl oder eine Gruppe der Formel II, wobei T ein Sauerstoffatom und R Phenyl oder Dichlorpyridyl bedeuten, sind.

Die Reste R¹ und R² der Verbindungen der Formel I können verschieden oder vorzugsweise gleich sein.

Besonders geeignet sind Cyanoguanidine, worin R¹ und R² jeweils Isopropyl, Allyl oder insbesondere Phenyl sind, sowie ein Cyanoguanidin der Formel I mit R¹ gleich Phenyl und R² gleich Allyl.

Die in den erfindungsgemässen Stoffgemischen enthaltenen Cyanoguanidine der Formel I können, wie gesagt, durch Umsetzung von Carbodiimiden der Formel III mit Cyanamid hergestellt werden. Die Umsetzung wird, falls zweckmässig, in Anwesenheit eines basischen Katalysators, z.B. eines tertiären Amins, wie Triethylamin, in einem inerten Lösungsmittel, wie z.B. 1,2-Dichlorethan, Diethylether, Tetrahydrofuran, Dioxan oder insbesondere in protischen Lösungsmitteln, wie z.B. Isopropanol, gegebenenfalls bei erhöhter Temperatur, durchgeführt.

Die Carbodiimide der Formel III sind bekannt und können auf bekannte Weise, zum Beispiel aus N,N′-disubstituierten Harnstoffen oder Thioharnstoffen oder aus Isocyanaten hergestellt werden. Geeignete Synthesemetho den sind beispielsweise von S.R. Sandler und W. Karo in "Organic Functional Group Preparation", Band 2 (Organic Chemistry Series Band 12-2), Academic Press, Orlando, FL, U.S.A. 1986, Seiten 233-258, beschrieben.

Selbstverständlich kann die Cyanoguanidinkomponente (b) der erfindungsgemässen Stoffgemische aus nur einer oder auch aus zwei oder mehr Verbindungen der Formel I bestehen. Wenn z.B. die Komponente (b) aus zwei oder mehr Verbindungen der Formel I besteht, können diese Cyanoguanidingemische entweder durch Vermischen von zwei oder mehr reinen Substanzen erhalten werden, oder auch als Gemische direkt hergestellt werden. Für die Herstellung solcher Gemische besonders geeignet ist z.B. die Umsetzung von zwei verschiedenen Isocyanaten R′-NCO und R˝-NCO zum entsprechenden Carbodiimidgemisch enthaltend R′-N=C=N-R′, R′-N=C=N-R˝ und R˝-N=C=N-R˝ gefolgt von einer Umsetzung des so erhaltenen Carbodiimidgemisches mit Cyanamid. Für bestimmte Anwendungen wird die Verwendung solcher Cyanoguanidingemische als Härter bevorzugt, da diese im allgemeinen eine bessere Löslichkeit in geeigneten Lösungsmitteln aufweisen.

Als Epoxidharze (a) der erfindungsgemässen Stoffgemische kommen alle diejenigen in Betracht, die mit den definitionsgemässen Cyanoguanidinen ausgehärtet werden können. Insbesondere seien genannt:
Alicyclische Polyepoxide, wie Epoxyethyl-3,4-epoxycyclohexan (Vinylcyclohexendiepoxid), Limonendiepoxid, Dicyclopentadiendiepoxid, Bis(3,4-epoxycyclohexylmethyl)adipat, 3′,4′-Epoxycyclohexylmethyl-3,4-epoxycyclohexancarboxylat, 3′,4′-Epoxy-6′-methylcyclohexylmethyl-3,4-epoxy-6-methylcyclohexancarboxylat, 3-(3′,4′-Epoxycyclohexyl)-2,4-dioxaspiro[5,5]-8,9-epoxyundecan, 3-Glycidyloxyethoxyethyl-2,4-dioxaspiro[5,5]-8,9-epoxyundecan.
Di- oder Polyglycidylether von mehrwertigen Alkoholen, wie 1,4-Butandiol oder Polyalkylenglykolen, wie Polypropylenglykole, Di- oder Polyglycidylether von cycloaliphatischen Polyolen, wie 2,2-Bis(4-hydroxycyclohexyl)propan, Di- oder Polyglycidylether von mehrwertigen Phenolen, wie Resorcin, Bis(p-hydroxyphenyl)methan (Bisphenol F), 2,2-Bis(p-hydroxyphenyl)propan (Bisphenol A), 2,2-Bis-(4′-hydroxy-3′,5′-dibromphenyl)propan, 1,1,2,2-Tetrakis(p-hydroxyphenyl)ethan, oder unter saueren Bedingungen erhaltene Kondensationsprodukte von Phenolen mit Formaldehyd, wie Phenol-Novolake und Kresol-Novolake; ferner Di- oder Poly(β-methylglycidyl)ether der oben angeführten Polyalkohole und Polyphenole.
Polyglycidylester und Poly(β-methylglycidyl)ester von mehrwertigen Carbonsäuren, wie Phthalsäure, Terephthalsäure, Tetrahydrophthalsäure und Hexahydrophthalsäure.
N-Glycidylderivate von Aminen, Amiden und heterocyclischen Stickstoffbasen, wie N,N-Diglycidylanilin, N,N-Diglycidyltoluidin, N,N,N′,N′-Tetraglycidyl-bis(p-aminophenyl)methan, Triglycidylisocyanurat, N,N′-Diglycidylethylenharnstoff, N,N′-Diglycidyl-5,5-dimethylhydantoin, N,N′-Diglycidyl-5-isopropyl-hydantoin, N,N′-Diglycidyl-5,5-dimethyl-6-isopropylo-5,6-dihydrouracil.

Bevorzugt als Epoxidharze (a) werden Polyglycidylderivate von Aromaten oder von Heteroaromaten eingesetzt, insbesondere Polyglycidylether von Bisphenolen, wie 2,2-Bis(4′-hydroxyphenyl)propan (Bisphenol A) oder Bis(4-hydroxyphenyl)methan (Bisphenol F).

Selbstverständlich können als Komponente (a) auch Gemische verschiedener Epoxidharze eingesetzt werden. Die Epoxidharz-Komponente (a) enthält vorzugsweise keine Epoxidharze, welche mit Elastomeren modifiziert sind.

Gewünschtenfalls kann man den härtbaren Gemischen zur Herabsetzung der Viskosität aktive Verdünner, wie z.B. Styroloxid, Butylglycidylether, 2,2,4-Trimethylpentylglycidylether, Phenylglycidylether, Kresylglycidylether, Glycidylester von synthetischen, hochverzweigten, in der Hauptsache tertiären aliphatischen Monocarbonsäuren zusetzen.

Die erfindungsgemässen härtbaren Gemische können zwar neben den Cyanoguanidinen der Formel I noch weitere übliche Härter für Epoxidharze enthalten, bevorzugt werden aber Gemische, welche keine zusätzlichen Härter aufweisen, besonders solche, welche keine Polyether-Polyamine enthalten.

Man kann bei der Härtung ausserdem Härtungsbeschleuniger einsetzen; solche Beschleuniger sind z.B. tertiäre Amine, deren Salze oder quaternäre Ammoniumverbindungen, z.B. Benzyldimethylamin, 2,4,6-Tris(dimethylaminomethyl)phenol, 1-Methylimidazol, 2-Ethyl-4-methylimidazol, N-Acylimidazole, wie z.B. die in den US 4,436,892 und US 4,587,311 beschriebenen Verbindungen, 4-Aminopyridin, Tripentylammoniumphenolat; oder Alkalimetallalkoholate, wie z.B. Natriumhexantriolat. Die erfindungsgemässen Stoffgemische enthalten vorzugsweise keine substituierten Guanidine als Härtungsbeschleuniger.

Bevorzugt werden erfindungsgemässe Stoffgemische, welche zusätzlich zu den Komponenten (a) und (b) noch (c) einen Härtungsbeschleuniger, vorzugsweise ein Imidazol-Derivat, enthalten.

Bei den in den erfindungsgemässen Stoffgemischen eingesetzten Komponenten (a), (b) und (c) kann es sich jeweils um Einzelverbindungen oder um Gemische handeln.

Die erfindungsgemässen Stoffgemische enthalten vorzugsweise 5-30 Gew.%, insbesondere 10-20 Gew.% des Cyanoguanidins (b) und gegebenenfalls 0,05 -5 Gew.%, vorzugsweise 0,1-1 Gew.%, des Beschleunigers (c) bezogen auf die Menge von (a) + (b).

Ein weiterer Gegenstand der Erfindung ist die Verwendung der erfindungsgemässen Stoffgemische zur Herstellung von vernetzten Produkten.

Die Härtung der erfindungsgemässen Mischungen wird zweckmässig im Temperaturintervall von 100°C bis 300°C, bevorzugt von 120 - 250°C, durchgeführt. Man kann die Härtung in bekannter Weise auch zwei-oder mehrstufig durchführen, wobei die erste Härtungsstufe bei niedriger Temperatur und die Nachhärtung bei höherer Temperatur durchgeführt wird.

Die Härtung kann gewünschtenfalls auch derart in 2 Stufen erfolgen, dass die Härtungsreaktion zunächst vorzeitig abgebrochen bzw. die erste Stufe bei wenig erhöhter Temperatur durchgeführt wird, wobei ein noch schmelzbares und/oder lösliches, härtbares Vorkondensat (sogenannte "B-Stufe") aus der Epoxid-Komponente (a) und dem Härter (b) erhalten wird. Ein derartiges Vorkondensat kann z.B. zur Herstellung von "Prepregs", Pressmassen oder Sinterpulvern dienen.

Der Ausdruck "Härten", wie er hier gebraucht wird, bedeutet die Umwandlung der löslichen, entweder flüssigen oder schmelzbaren Polyepoxide in feste, unlösliche und unschmelzbare, dreidimensional vernetzte Produkte bzw. Werkstoffe, und zwar in der Regel unter gleichzeitiger Formgebung zu Formkörpern, wie Giesskörpern, Presskörpern und Schichtstoffen, zu Imprägnierungen, Beschichtungen, Lackfilmen oder Verklebungen.

Die erfindungsgemässen Stoffgemische eignen sich insbesondere als Laminierharze für die Herstellung von Prepregs und faserverstärkten Verbundstoffen.

Die nachfolgenden Beispiele erläutern die Erfindung näher.

### Beispiel 1: N-Cyano-N′-(2,6-diisopropyl-4-phenoxyphenyl)-N˝-tert.-butylguanidin

Zur Lösung von 3,5 g N-(2,6-Diisopropyl-4-phenoxyphenyl)-N′-tert.-butylcarbodiimid [hergestellt gemäss Beispiel 1 (Verbindung Nr. 4) der EP-A 175 649] in 20 ml 1,2-Dichlorethan werden 0,46 g Cyanamid gegeben, und es wird 21 Std. am Rückfluss erwärmt. Das Reaktionsgemisch wird bis zu einer konzentrierten Suspension eingedampft, mit Ethylether verrührt, und das kristalline Produkt wird abgenutscht; Smp. 233,5 - 235,0°C (aus Diethylether-Hexan).

### Beispiel 2: N-Cyano-N′-[2,6-dimethyl-4-(3′,5′-dichlor-2′-pyridyloxy)phenyl]-N˝ -tert.-butylguanidin

Es werden 4,26 g N-[2,6-Dimethyl-4-(3′,5′-dichlor-2′-pyridyloxy)-phenyl]-N′-tert.-butylcarbodiimid [hergestellt gemäss Beispiel 1 (Verbindung Nr. 149) der EP-A 175 649] in 20 ml 1,2-Dichlorethan mit 0,54 g Cyanamid wie im Beispiel 1 beschrieben umgesetzt und aufgearbeitet. Man erhält das Produkt als farblose Kristalle mit einem Smp. von 206 - 207°C.

### Beispiel 3: N-Cyano-N′-cyclohexyl-N˝-phenylguanidin

1,8 g N-Cyclohexyl-N′-phenylcarbodiimid [hergestellt gemäss Tetrahedron Lett. 26, 1661 (1985)] werden in 20 ml 1,2-Dichlorethan mit 0,42 g Cyanamid wie in Beispiel 1 beschrieben umgesetzt und aufgearbeitet. Das ölig-viskose Rohprodukt wird an Kieselgel mit Methylenchlorid-Diethylether 4:1 chromatographiert, und man erhält das kristalline Produkt mit einem Smp. von 147 - 147,5°C (Essigester).

### Beispiel 4: N-Cyano-N′-2-methoxycarbonyl-ethyl-N˝-phenylguanidin

Zur Lösung von 0,75 g N-2-Methoxycarbonylethyl-N′-phenylcarbodiimid in 8 ml 1,2-Dichlorethan werden 0,17 g Cyanamid gegeben, und es wird 5 Std. am Rückfluss erwärmt. Das Reaktionsgemisch wird am Vakuum eingeengt und der Rest wird an Kieselgel mit Methylenchlorid-Diethylether-Essigester 1:1:1 chromatographiert. Man erhält das farblose kristalline Produkt mit einem Smp. von 122,5 - 123,5°C.

### N-2-Methoxycarbonyl-ethyl-N′-phenylcarbodiimid:

Zur Lösung von 0,95 g N-2-Methoxycarbonyl-ethyl-N′-phenylthioharnstoff und 0,97 g Triethylamin in 20 ml Acetonitril werden bei Raumtemperatur 1,2 g 2-Chlor-1-methylpyridiniumjodid gegeben. Es wird 1 Std. gerührt, die entstandene Suspension wird eingedampft, in Pentan aufgenommen und mit Wasser und Sole gewaschen. Nach dem Trocknen mit Magnesiumsulfat, Filtrieren und Einengen am Vakuum erhält man das Produkt als schwach gelbliches Oel.

### Beispiel 5: N-Allyl-N′-cyano-N˝-phenylguanidin

0,6 g N-Allyl-N′-phenylcarbodiimid werden in 8 ml 1,2-Dichlorethan mit 0,17 g Cyanamid wie im Beispiel 4 beschrieben umgesetzt und aufgearbeitet. Umkristallisation aus Diethylether liefert das farblose kristalline Produkt mit einem Smp. von 99,5 - 100,5°C.

### N-Allyl-N′-phenylcarbodiimid:

0,77 g N-Allyl-N′-phenylthioharnstoff, 0,97 g Triethylamin und 1,2 g 2-Chlor-1-methylpyridiniumiodid werden wie im Beispiel 4 beschrieben umgesetzt und aufgearbeitet. Man erhält das Produkt als hellgelbes Oel.

### Beispiel 6: N,N′-Bisallyl-N˝-cyanoguanidin

0,7 g N,N′-Bisallylcarbodiimid werden in 10 ml 1,2-Dichlorethan mit 0,27 g Cyanamid wie im Beispiel 4 beschrieben umgesetzt und aufgearbeitet. Umkristallisation aus Diethylether ergibt das farblose kristalline Produkt mit einem Smp. von 66,5 - 67,0°C.

### N,N′-Bisallylcarbodiimid:

1,56 g N,N′-Bisallylthioharnstoff, 2,4 g Triethylamin und 3,07 g 2-Chlor-1-methylpyridiniumiodid werden in 40 ml Acetonitril wie im Beispiel 4 beschrieben umgesetzt und aufgearbeitet. Man erhält das Produkt als schwach gelbliches Oel.

### Beispiel 7: N,N′-Bis(1-naphthyl)-N˝-cyanoguanidin

Einen 500 ml Dreihalsrundkolben, versehen mit Rührer, Thermometer, Rückflusskühler sowie Trockenrohr und Blasenzähler beschickt man mit:
126,9 g (0,75 Mol) 1-Naphthylisocyanat
0,5 g (2,60 mMol) 3-Methyl-1-phenyl-1-phospha-3-cyclopenten-1-oxid und
200 ml Toluol.
Diese Lösung rührt man 2 h unter Rückfluss. Dabei entwickelt sich CO₂. Danach entfernt man das Lösungsmittel am Rotationsverdampfer. Zum Rückstand (gelbliches Oel) gibt man:
250 ml Isopropanol
10 ml Triethylamin
31,5 g (0,75 Mol) Cyanamid (portionsweise in 15 Min.).
Das Reaktionsgemisch wird während 2h bei 70 - 80°C gerührt. Danach kühlt man mit einem Eisbad auf ca. 10°C und filtriert den Niederschlag ab. Nach dem Waschen mit Isopropanol und Trocknen im Vakuum verbleiben 124 g (98 % d. Th.) farbloses Pulver mit einem Schmelzpunkt von 224 - 225°C.

### Elementaranalyse:

berechnet (%) :
C 78,5 H 4,8 N 16,7
gefunden :
C 77,7 H 4,9 N 17,4

### Beispiele 8 - 12:

Nach dem in Beispiel 7 beschriebenen Verfahren werden die folgenden Verbindungen hergestellt:

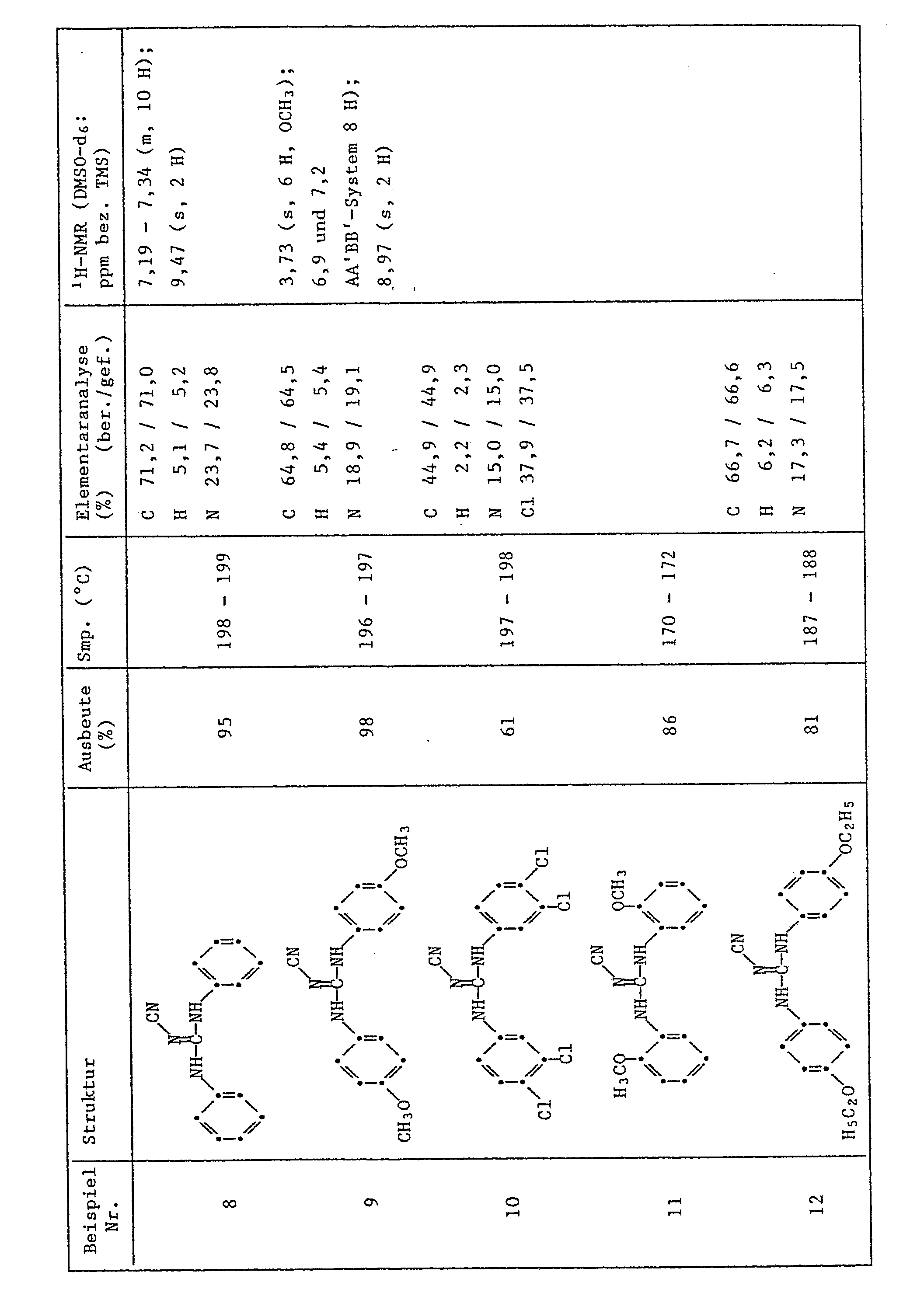

### Beispiel 13: N,N′-Biscyclohexyl-N˝-cyanoguanidin

Einen 1,5 l Sulfierkolben, versehen mit Rührer, Thermometer, Rückflusskühler und Trockenrohr beschickt man mit:
206,2 g (1,0 Mol) N,N′-Dicyclohexylcarbodiimid
500 ml Isopropanol und
10 ml Triethylamin.
Dazu gibt man bei Raumtemperatur unter Rühren portionsweise in ca. 15 Min. 84,1 g (2,0 Mol) Cyanamid. Anschliessend wird das Reaktionsgemisch während 2h bei 70 - 80°C gerührt. Danach wird mit einem Eisbad auf ca. 10°C gekühlt und der Niederschlag wird abfiltriert. Nach dem Waschen mit Isopropanol und Trocknen im Vakuum verbleiben 190 g (77 % d. Th.) farblose Kristalle mit einem Schmelzpunkt von 191°C.

### Elementaranalyse:

berechnet (%):
C 67,7 H 9,7 N 22,6
gefunden :
C 67,7 H 9,7 N 22,6

### Beispiele 14 - 17:

Nach dem in Beispiel 13 beschriebenen Verfahren werden die in der Tabelle aufgeführten Verbindungen hergestellt. Die entsprechenden Carbodiimide sind bekannt und können auf bekannte Weise hergestellt werden.

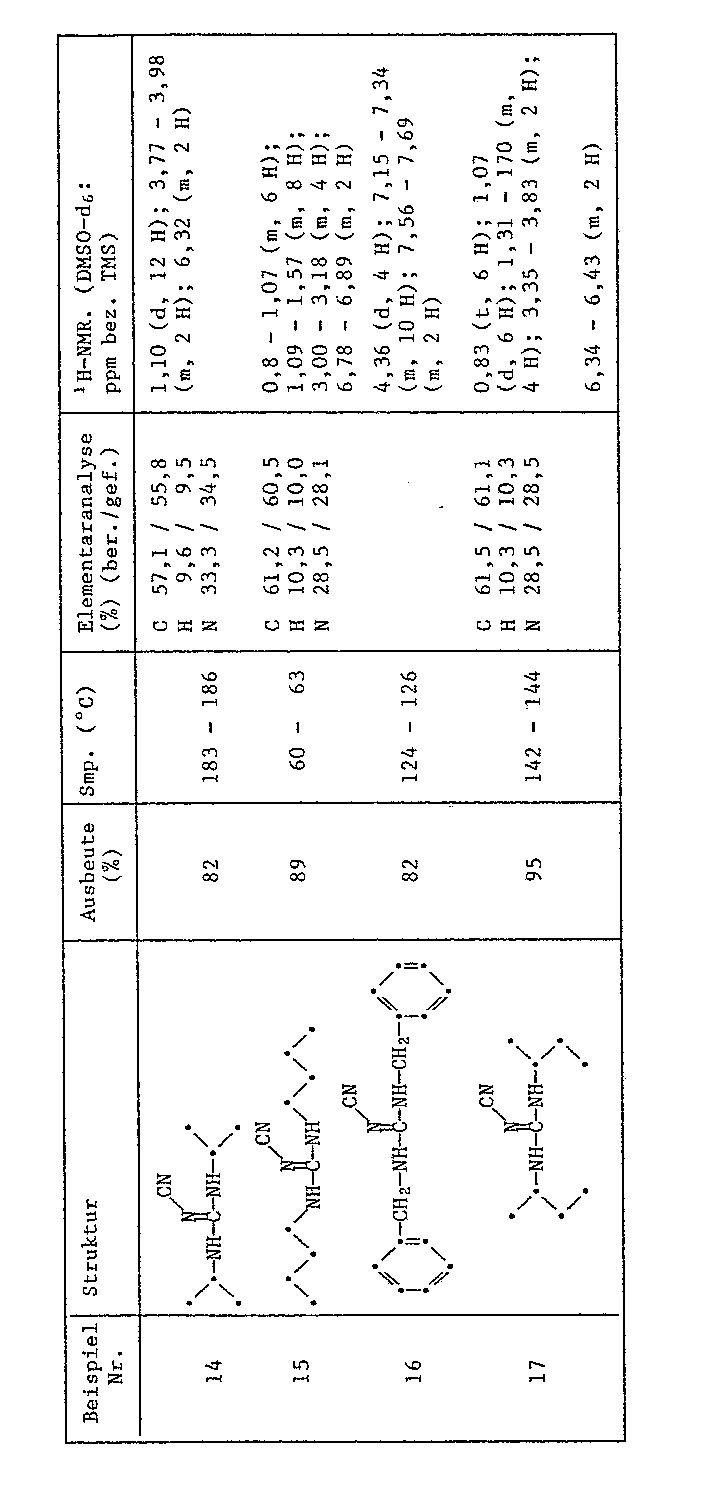

### Beispiel 18: Herstellung eines Cyanoguanidingemisches ausgehend von äquimolaren Mengen von Phenylisocyanat und 4-Methoxyphenylisocyanat

Aequimolare Mengen von Phenylisocyanat und 4-Methoxyphenylisocyanat werden gemäss dem in Beispiel 7 beschriebenen Verfahren zum entsprechenden Cyanoguanidingemisch umgesetzt. Das so erhaltene Produkt hat einen Schmelzbereich von 150 - 158°C.

### Anwendungsbeispiele

Mit den in der Tabelle angegebenen Mengen der Cyanoguanidine werden mit Bisphenol-A-diglycidylether (Epoxidgehalt 5,4 Aequivalente/kg) jeweils 100 g Mischung hergestellt und 4 h bei 180°C gehärtet. Dabei entstehen klare Giesskörper mit der in der Tabelle angegebenen Glasumwandlungstemperatur (Tg, ermittelt mittels DSC).

| Anwendungsbeispiel | Cyanoguanidin gemäss Beispiel Nr. (g) | Tg (°C) |
|---|---|---|
| A₁ | 7 (20) | 145 |
| A₂ | 8 (15) | 145 |
| A₃ | 9 (20) | 139 |
| A₄ | 13 (15) | 129 |
| A₅ | 14 (10) | 140 |
| A₆ | 18 (20) | 130 |
| A₇ | 1 (20) | 125 |
| A₈ | 2 (20) | 92 |
| A₉ | 3 (15) | 110 |
| A₁₀ | 4 (15) | 122 |
| A₁₁ | 5 (15) | 116 |
| A₁₂ | 6 (10) | 90 |

## Patentansprüche

1. Härtbares Stoffgemisch enthaltend
(a) ein Epoxidharz und
(b) als Härter für das Epoxidharz ein Cyanoguanidin der Formel I 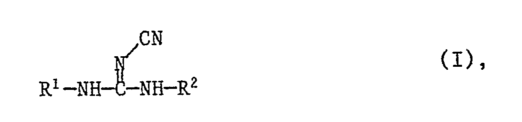 worin R¹ und R² unabhängig voneinander C₁-C₁₂-Alkyl, C₂-C₁₂-Alkenyl, C₅-C₁₀-Cycloalkyl, C₆-C₁₀-Aryl, C₇-C₁₂-Aralkyl sind, oder einen C₄-C₈-heterocyclischen Rest oder eine Gruppe der Formel II bedeuten 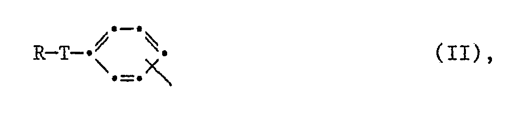 worin R für Phenyl oder für einen C₄-C₅-heterocyclischen Rest und T für Methylen, Isopropyliden, CO, O, S oder SO₂ stehen, wobei die Reste R¹ und R² unsubstituiert sind oder C₁-C₄-Alkyl, C₁-C₄-Alkoxy, Nitro, Halogen,
R³-O- 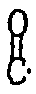 - oder
R³- 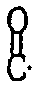 -O- Substituenten enthalten und R³ Phenyl oder C₁-C₄-Alkyl bedeutet.

2. Stoffgemisch nach Anspruch 1, worin R¹ und R² unabhängig voneinander C₁-C₆-Alkyl, C₂-C₆-Alkenyl, C₅-C₆-Cycloalkyl, C₆-C₁₀-Aryl, Benzyl oder einen Rest der Formel II bedeuten.

3. Stoffgemisch nach Anspruch 1, worin R¹ und R² unabhängig voneinander C₃-C₄-Alkyl, C₃-C₄-Alkenyl, Cyclohexyl, Phenyl, Tolyl, Methoxyphenyl, Ethoxyphenyl, Dichlorphenyl, Benzyl, Naphthyl oder eine Gruppe der Formel II, wobei T ein Sauerstoffatom und R Phenyl oder Dichlorpyridyl bedeuten, sind.

4. Stoffgemisch nach Anspruch 1, worin R¹ und R² gleich sind.

5. Stoffgemisch nach Anspruch 4, worin R¹ und R² Phenyl, Isopropyl oder Allyl sind.

6. Stoffgemisch nach Anspruch 1, worin R¹ Phenyl und R² Allyl sind.

7. Stoffgemisch nach Anspruch 1, worin die Cyanoguanidinkomponente (b) ein Gemisch aus zwei oder mehr Verbindungen der Formel I ist.

8. Stoffgemisch nach Anspruch 1, worin das Epoxidharz (a) ein Polyglycidylderivat eines Aromaten oder eines Heteroaromaten ist.

9. Stoffgemisch nach Anspruch 8, worin die Komponente (a) ein Polyglycidylether von 2,2-Bis(4′-hydroxyphenyl)propan oder von Bis(4-hydroxyphenyl)methan ist.

10. Stoffgemisch nach Anspruch 1, enthaltend zusätzlich (c) einen Härtungsbeschleuniger.

11. Stoffgemisch nach den Ansprüchen 1 oder 10, enthaltend 5-30 Gew.% des Cyanoguanidins (b) und gegebenenfalls 0,05-5 Gew.% des Beschleunigers (c), bezogen auf die Menge von (a) + (b).

12. Verwendung des Stoffgemisches nach Anspruch 1 zur Herstellung von vernetzten Produkten.

13. Verwendung des Stoffgemisches nach Anspruch 1 als Laminierharz für die Herstellung von Prepregs und faserverstärkten Verbundstoffen.
